# FASCICULE DE BREVET EUROPEEN

(11) **EP 3 247 296 B1**
(45) Date de publication et mention de la délivrance du brevet: **23.03.2022**
(21) Numéro de dépôt: 16703584.9
(22) Date de dépôt: 19.01.2016
(51) Int. Cl.: A61B 17/70, A61B 17/82, A61B 17/84

(54) **DISPOSITIF DE FIXATION D'UNE BANDE PLATE SUR UNE PARTIE OSSEUSE**
VORRICHTUNG ZUR BEFESTIGUNG EINES FLACHBANDES AN EIN KNOCHENTEIL
DEVICE FOR ATTACHING A FLAT BAND ON A BONE PART

(30) Priorité: 20.01.2015 FR 1550441
(43) Date de publication de la demande: 29.11.2017
(73) Titulaire: Implanet, 33650 Martillac (FR)
(72) Inventeur: PASQUET, Denis, 13100 Aix En Provence (FR); LE COUËDIC, Régis, 33000 Bordeaux (FR)
(74) Mandataire: Benech, Frédéric
(86) Numéro de dépôt international: PCT/FR2016/050096
(87) Numéro de publication internationale: WO 2016/116692

(56) Documents cités:
- EP-A1- 2 316 363
- US-A- 4 455 717
- US-A- 5 356 412
- US-A- 5 383 905
- US-B1- 6 589 246

## Description

La présente invention concerne un dispositif de fixation d'une bande plate et souple sur au moins une partie osseuse.

Elle trouve une application particulièrement importante bien que non exclusive dans le domaine de la consolidation de la colonne vertébrale et notamment de la solidarisation de deux vertèbres entre elles en cas de lésions.

On sait que les disques intervertébraux peuvent être l'objet de tassement, d'hernie discale ou de dégénérescence arthrosique intervertébrale.

Pour soulager les patients, il existe alors des techniques nécessitant une intervention chirurgicale.

Une première technique consiste à remplacer le disque endommagé par une prothèse de disque intervertébral. Un tel remplacement est délicat à effectuer par le chirurgien et présente un risque de désolidarisation sous l'effet des forces de cisaillements importantes qui s'exercent notamment lorsque la prothèse est en position de flexion maximale.

Une deuxième technique consiste à réaliser une arthrodèse intervertébrale opération permettant de fusionner les deux vertèbres adjacentes au disque endommagé. Il y a alors blocage de la dégradation du fait de la suppression de toute mobilité entre les deux vertèbres concernées.

Une telle technique implique l'utilisation d'un dispositif de stabilisation des deux vertèbres comportant en général des vis destinées à être vissées dans les vertèbres et solidarisées entre elles par un organe rigide de liaison. Il est ainsi possible d'éviter que des contraintes mécaniques trop importantes ne soient appliquées au disque intervertébral.

Ici encore il existe des inconvénients nécessitant en particulier de visser dans les vertèbres ce qui est une opération structurellement agressive fragilisant les vertèbres concernées, voire impossible si ces dernières sont en mauvais état et/ou insuffisamment larges au niveau de la fixation.

Une autre technique consiste à utiliser des crochets. Mais cette utilisation est quant à elle très délicate puisque l'opérateur ne doit pas toucher la moelle épinière sous peine de paralysie du patient. Il y a de plus des risques de glissement et/ou de décrochement.

On connait également des dispositifs de fixation pour fil de suture (US 5,383,905) ou pour bande plate (EP 2 316 363) par coincement d'une pièce conique dans un évidement de forme complémentaire.

Ces dispositifs ne sont pas faciles à mettre en place compte tenu de leur petitesse. D'autres éléments (US 4,455,717) réalisent une fixation du lien par vissage puis ancrage par picots internes agencés pour percer le lien. Un tel système, outre qu'il est agressif ne permet pas l'ajustage.

La présente invention vise à fournir un dispositif de fixation sur une partie osseuse répondant mieux que ceux antérieurement connus aux exigences de la pratique, notamment en ce qu'elle n'est pas limitée à la fixation d'au moins deux parties osseuses entre elles, en ce qu'elle permet une plus grande flexibilité, en ce qu'elle évite tout élément intrusif dans l'os, en ce qu'elle présente une absence de risque de glissement et/ou de perte de tension sur les vertèbres, et ce pour un coût faible et/ou moindre qu'avec les dispositifs de l'art antérieur et en ce qu'elle permet une préparation de la connexion entre corps de base et pièce de maintien.

Elle présente également l'avantage d'un maniement aisé de la bande souple, permettant sa mise en place facile par le chirurgien autour des parties osseuses.

Dans ce but l'invention propose notamment un dispositif de fixation d'une bande plate et souple sur au moins une partie osseuse, comprenant ladite bande présentant deux portions d'extrémité, un corps de base rigide comportant un orifice traversant, et une pièce de maintien insérable dans ledit orifice, caractérisé en ce que la pièce de maintien est au moins en partie en forme de coin écrasable comprenant un alésage central de passage desdites portions d'extrémité en vis à vis, ledit alésage étant de section transversale déformable entre une première section de passage libre des portions d'extrémité lorsque la pièce n'a pas été insérée dans l'orifice et une deuxième section de blocage desdites portions d'extrémité en compression lorsque la pièce est entièrement ou sensiblement entièrement insérée dans l'orifice.

Dans des modes de réalisation avantageux, on de plus recours à l'une et/ou à l'autre des dispositions suivantes :
- le corps de base étant allongé autour d'un axe, l'orifice présente une première section longitudinale trapézoïdale ou sensiblement trapézoïdale isocèle dans un premier plan axial avec un premier angle à la base a et la pièce de maintien une seconde section longitudinale trapézoïdale ou sensiblement trapézoïdale isocèle dans ledit premier plan axial avec un second angle à la base b > a.

Par sensiblement trapézoïdale on entend une section de forme géométrique dont les côtés latéraux du trapèze présentent une périphérie s'inscrivant dans un trapèze mais ne sont pas forcément linéaires ou formés par une ligne droite (en étant dentelé par exemple) ;
- la pièce de maintien comprend deux parois longitudinales opposées d'extrémité chacune munie d'une fente traversante sur une portion de ladite paroi ouvrant sur l'alésage et débouchant sur la face supérieure de la pièce.

Les fentes ou saignées favorisent l'écrasement de la pièce lors de son introduction en force dans l'orifice, tout en permettant au départ un alésage aussi large que possible, ce qui facilite l'introduction et le passage des extrémités de la bande avant compression.
- la face interne de l'orifice et la face périphérique externe de la pièce comprennent des moyens de pré connexion de l'un à l'autre par encliquetage.

Ces moyens de pré connexion jouent un rôle anti-retour permettant un pré-montage du dispositif, évitant aux différents éléments du dispositif de se désolidariser par exemple dans un emballage de transport, et dès lors d'avoir à être recherchés et réassemblés par le chirurgien au moment de son intervention ;
- la face interne de l'orifice du corps et la face périphérique externe de la pièce comportent des moyens en vis-à-vis constituant un système de blocage de la pièce par rapport au corps, lorsque la pièce est insérée dans l'orifice.
- l'alésage et la pièce présentent chacun un plan de symétrie longitudinal définissant respectivement deux faces longitudinales internes opposées pour l'orifice et deux faces longitudinales périphériques externes opposées pour la pièce, chaque face opposée comportant au moins une portion munie d'au moins une dent ou nervure, d'accrochage et de blocage de la pièce par rapport au corps lorsque la pièce est insérée dans l'orifice.

Ces moyens d'accrochage autorisent un prépositionnement dans l'alignement des éléments les uns par rapport aux autres, ce qui permettra leur mise en compression de façon simple par une pince ancillaire de préhension de conception à la portée de l'homme du métier.

Ils constituent également un système anti-retour évitant la désolidarisation de la pièce et du corps, lorsque la pièce est insérée dans le corps.
- Les faces longitudinales périphériques externes de la pièce sont au moins en partie en biais par rapport au plan de symétrie pour donner la forme de coin ;
- l'alésage présente une section transversale de forme oblongue et comporte deux parois principales opposées, au moins une paroi principale comprenant sur sa face interne au moins une nervure transversale en saillie vers l'intérieur. Avantageusement, elle en comporte deux en vis à vis, décalées en hauteur ;
- la nervure transversale en saillie est située dans la partie longitudinale supérieure de l'alésage;
- l'alésage et la pièce de maintien sont de formes agencées pour coopérer à frottement sur au moins une de leur partie en vis à vis dans le sens perpendiculaire au premier plan longitudinal lorsque la pièce est insérée dans l'orifice;
- la pièce présente un noyau évidé par l'alésage, de forme générale oblongue, comprenant deux parois principales opposées en biais formant le coin et deux parois d'extrémité allonges autour d'un axe, arrondies, dont les faces sont agencées pour coopérer longitudinalement avec une partie en évidement correspondante de l'orifice, l'épaisseur des jonctions entre parois principales et parois d'extrémité étant amoindrie et agencée pour autoriser la déformation par écrasement dans le sens transversal sans déformation dans le sens longitudinal lors de l'insertion de la pièce dans l'orifice.

Une telle disposition permet à la pièce de se déformer dans un plan, dans le sens du coincement recherché, tout en gardant une excellente capacité de guidage sans déformation/dilatation dans l'autre plan, perpendiculaire au premier.

Avantageusement les parois d'extrémité allongées autour d'un axe sont chacune munie d'au moins une fente transversale sur une partie de sa hauteur favorisant l'écrasement ;
- le corps de base est en titane, la pièce de maintien est en matériau polymère et la bande souple est une tresse en polymère.

La fixation de la bande plate et souple sur au moins une partie osseuse, ladite bande étant munie de deux portions d'extrémité, se fait ainsi via un corps de base rigide comportant un orifice traversant et une pièce de maintien insérable dans ledit orifice. La pièce de maintien étant en forme de coin écrasable comprenant un alésage central de passage desdites portions d'extrémité en vis à vis de la bande souple, on introduit une portion d'extrémité de la bande dans l'orifice et dans l'alésage, la pièce étant connectée partiellement(préalablement ou postérieurement) dans l'orifice, on passe la bande autour de la partie osseuse, on passe l'autre portion d'extrémité dans l'orifice et dans l'alésage, formant ainsi une boucle de préhension de ladite partie osseuse, on serre la boucle à la dimension voulue en tirant sur les deux portions d'extrémité, et on insère en force la dite pièce dans l'orifice en déformant l'alésage pour obtenir une position de blocage desdites portions d'extrémité en compression dans l'alésage, la pièce étant alors déformée plastiquement.

Avantageusement on solidarise entre elles au moins deux vertèbres adjacentes dans le cas d'une opération d'arthrodèse.

Pour ce faire, on passe une portion d'extrémité de la bande dans le corps de base et dans la pièce, on passe la bande plate autour des deux vertèbres (on forme une boucle avec le reste de la bande autour des vertèbres) et on passe l'autre portion d'extrémité dans l'orifice et l'alésage, on rapproche les vertèbres en tirant sur les extrémités et on verrouille la fixation en insérant en force la pièce dans le corps dans la position voulue avant de couper les parties d'extrémités en surplus de la bande.

L'invention sera mieux comprise à la lecture de la description qui suit de modes de réalisation donnés ci-après à titre d'exemple non limitatifs.

La description se réfère aux dessins qui l'accompagnent dans lesquels :
Les figures 1A et 1B sont des vues en perspective d'un dispositif selon un mode de réalisation de l'invention, avant insertion (figure 1A) et après insertion complète (figure 1B) de la pièce de maintien dans le corps de base.
La figure 2 est une vue en perspective de la pièce de maintien de la figure 1.
Les figures 2A, 2B et 2C sont respectivement la coupe IIA-IIA de la figure 2 (figure 2A), et les vues de dessus avant insertion (figure 2B) et après insertion (figure 2C) de la pièce de la figure 2.
La figure 3 est une vue en coupe axiale longitudinale d'une pièce selon un autre mode de réalisation de l'invention.
La figure 4 est une vue en perspective du corps de base de la figure 1.
La figure 4A est une vue en coupe selon IVA-IVA de la figure 4.
La figure 4B est une vue de dessus du corps de la figure 4.
La figure 5 montre schématiquement et de profil un dispositif selon la figure 1 en place sur deux vertèbres adjacentes.
La figure 6 est une vue schématique en perspective et par le dessus, montrant une pièce avec fente selon un autre mode de réalisation de l'invention.

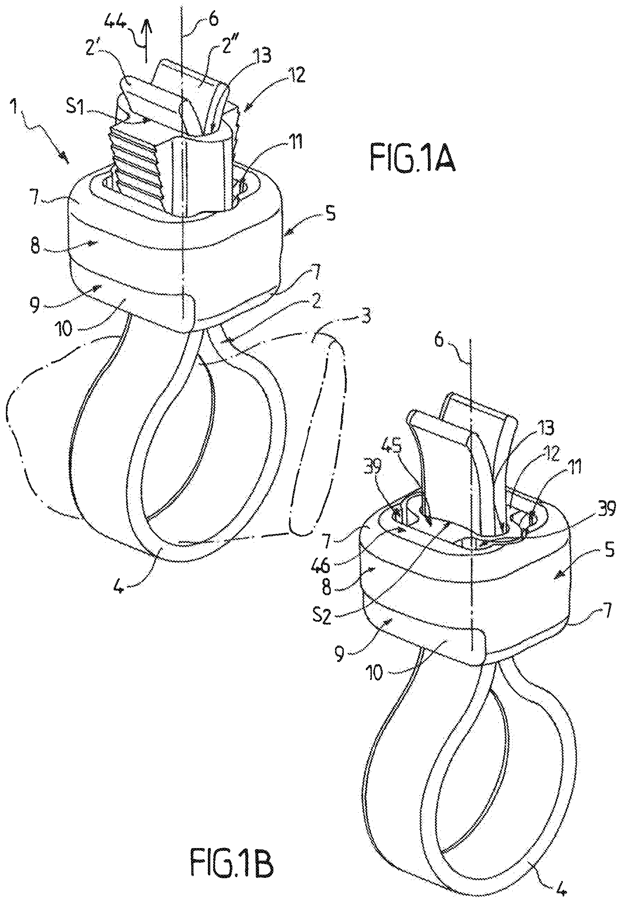

Les figures 1A et 1B montrent un dispositif 1 de fixation d'une bande 2 plate et souple sur au moins une partie osseuse 3.

La bande 2 est par exemple en polyester tressé, par exemple en polyester de 1 à 3 mm d'épaisseur et de 6 mm de large et présente une boucle 4 de fixation sur la partie osseuse 3, par exemple sur une branche transverse ou épineuse d'une vertèbre.

La boucle 4 est formée par le rapprochement des deux portions d'extrémités 2' et 2" de la bande.

Le dispositif 1 comprend un corps 5 de base rigide, par exemple en titane, mais il peut également être en matière plastique rigide.

Le corps 5 est allongé autour d'un axe 6. Il est de forme sensiblement parallélépipédique ou cubique à bords 7 par exemple arrondis par exemple avec une partie supérieure 8 élargie et une partie inférieure 9 rétrécie transversalement en présentant deux évidements latéraux 10 qui vont permettre sa préhension par les mâchoires d'une pince ancillaire.

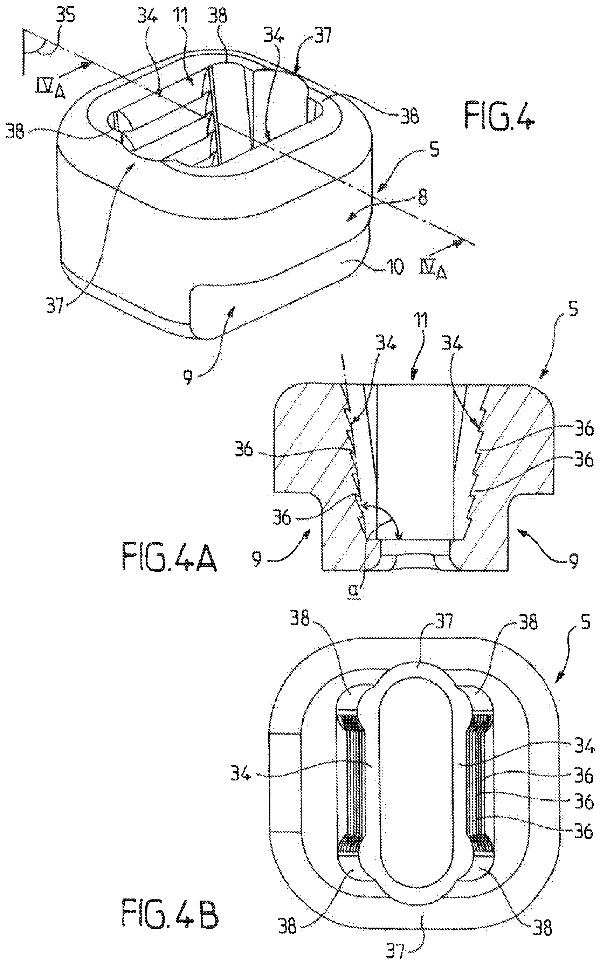

Il est muni d'un orifice traversant 11 de section sensiblement oblongue et qui sera détaillé en référence aux figures 4 à 4B.

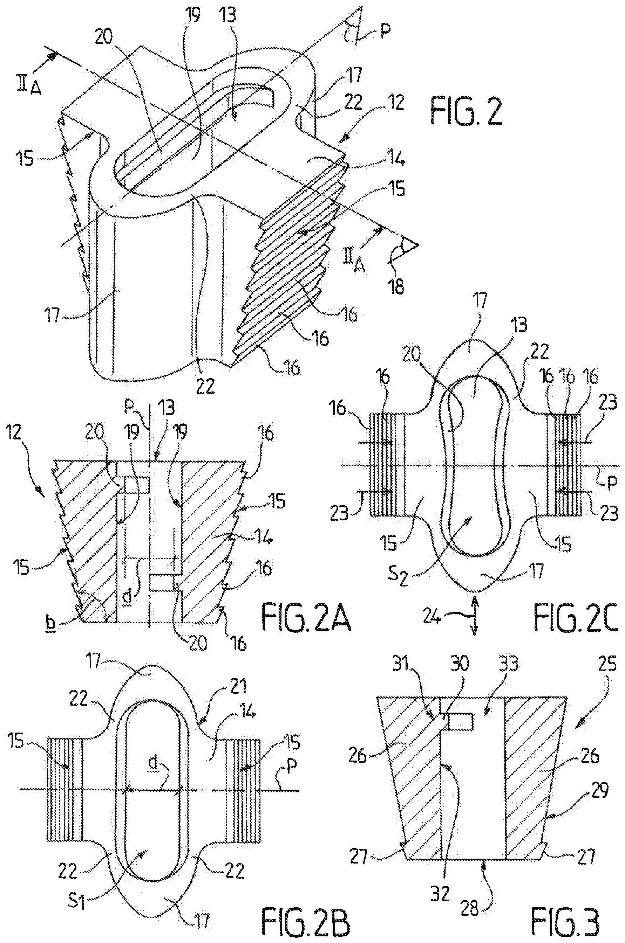

Le dispositif 1 comprend de plus une pièce 12 de maintien de la bande 2, par exemple en matière plastique, au moins en partie en forme de coin, c'est à dire de forme prismatique ou sensiblement prismatique qui sera détaillée en référence aux figures 2 et 2A à 2C.

La pièce 12 est percée d'un alésage 13 de passage des extrémités 2', 2" de la bande, de section transversale déformable entre une première section S1 de passage libre des portions d'extrémité lorsque la pièce 12 n'est pas insérée en compression réelle dans l'orifice (figure 1A) et une section S₂, rétrécie, de blocage des portions d'extrémité en compression lorsque la pièce est entièrement insérée dans l'orifice 11 (figure 1B).

En référence aux figures 2, la pièce 12 présente une première partie centrale 14 de section horizontale rectangulaire ou sensiblement rectangulaire décroissante, et de section verticale longitudinale (selon le plan de coupe II_{A}-II_{A}) de forme trapézoïdale ou sensiblement trapézoïdale isocèle à parois 15 symétriques par rapport au plan longitudinal P, à la périphérie dentelée régulièrement extérieurement (dents 16).

Les dents 16 (par exemple au nombre de sept) sont formées par des arêtes longitudinales en triangle, dont les sommets sont dirigés vers le haut ou sont horizontaux, à angle aigu (en écaille).

Les dents inférieures ou la dent inférieure de la pièce et les ou la dent supérieure(s) de l'orifice du corps, forment un moyen de pré-connexion de l'un par rapport à l'autre par encliquetage.

La pièce 12 présente de plus deux parties d'extrémité 17 en forme de demi cylindre ou sensiblement de demi-cylindre présentant des parois de section transversale ovale, symétriques par rapport au plan de coupe II_{A}-II_{A} 18, dont l'ovalite est dirigée vers l'extérieur et est agencée, comme on va le voir pour coopérer avec des parois en vis à vis de l'orifice 11.

Comme l'illustrent les figures 2 et 2B, les parois 15 et 17 de la pièce 12 sont pleines et ne comportent pas de discontinuité.

La section trapézoïdale présente un angle à la base b (par rapport à la petite base du trapèze).

L'angle entre les parois 15 (correspondant à 2b - 180°) est, de préférence compris entre 10 et 30°.

Plus précisément en référence aux figures 2B et 2C, l'alésage 13 présente une section transversale horizontale de forme oblongue, en langue de chat qui va se déformer entre une forme de section S1 (figure 2B) et une forme à milieu rétréci de section S2 (figure 2C).

L'alésage 13 comprend deux parois principales planes opposées 19 comprenant sur leur face interne une nervure 20 transversale décalée en hauteur l'une par rapport à l'autre, de section carrée ou rectangulaire se terminant en biseau affiné à ses extrémités, qui vont permettre un meilleur blocage des extrémités 2', 2" après insertion, la distance d entre les deux bords d'extrémités 19 des membres avant compression autorisant un passage libre desdites extrémités de la bande 2.

Les dimensions de l'alésage 13 sont, de manière générale, adaptées à celles de la bande.

En particulier, la distance d comme la longueur de l'alésage selon le plan de coupe P sont choisies pour que les deux extrémités de la bande soient bien superposées à plat. En effet, tout décalage entre les extrémités de la bande nuit à un bon serrage de la bande dans le dispositif 1.

En pratique, la distance d est légèrement supérieure à deux fois l'épaisseur de la bande et la longueur de l'alésage est légèrement supérieure à la largeur de la bande.

Dans le mode de réalisation plus particulièrement décrit ici la pièce 12 présente donc un noyau 21 évidé par l'alésage 13 formé par la première partie centrale 14 et les parties d'extrémités 17.

Il présente les parois principales opposées 15 dont la face externe est en biais et forme le coin relié par les deux parois arrondies d'extrémité 17 agencées pour coopérer à frottement avec l'orifice qui sera décrit ci-après.

L'épaisseur e des jonctions 22 entre parois principales et parois d'extrémité 17 est plus faible (par exemple deux fois moins épaisse) pour autoriser la déformation par écrasement dans le sens transversal (flèche 23) sans déformation dans le sens longitudinal (flèche 24) lors de l'insertion de la pièce dans l'orifice (cf. figure 2C).

On a représenté sur la figure 3 en coupe, un autre mode de réalisation d'une pièce 25 dont les parois principales 26 ne comportent qu'une dent 27 à l'extrémité basse 28 (petite base du trapèze) sur leur face externe 29 et une seule nervure 30, en partie haute 31 (9/10^{ème} de la hauteur par exemple) sur leur face interne 32 de l'alésage 33.

Les figures 4, 4A et 4B montrent le corps 5 de base utilisé avec la pièce 12.

Il est sensiblement parallélépipédique et comporte l'orifice traversant 11.

Celui-ci comporte deux portions longitudinales principales internes 34 symétriques par rapport au plan axial perpendiculaire au plan 35 (plan axial de la coupe IVA - IVA) en présentant une section longitudinale trapézoïdale d'angle à la base a, avec des dents 36 ou évidements de formes complémentaires à celles des faces externes des parois 15 de la pièce, en nombre identique ou similaire (cf. figure 4A) .

L'invention n'est pas limitée à ce mode de réalisation et couvre tous les moyens de formes complémentaires prévus sur la face interne de l'orifice 11 et sur la face externe de la pièce 12 permettant d'assurer la pré-connexion de la pièce et du corps ou encore un blocage de pièce dans le corps.

L'orifice comporte par ailleurs deux parois d'extrémité 37 agencées pour coopérer au moins en partie avec la face externe des parois 17 de la pièce, en étant de forme complémentaire, c'est à dire dans le mode de réalisation plus particulièrement décrit, de section interne demi-circulaire ou demi-ovale.

Toujours dans ce mode de réalisation décrit, les parois d'extrémité sont rattachées aux parois principales par des parois intermédiaires 38 de forme courbe et permettant de rattraper les épaisseurs de parois, qui vont laisser des évidements 39 (voir figure 1B) après insertion.

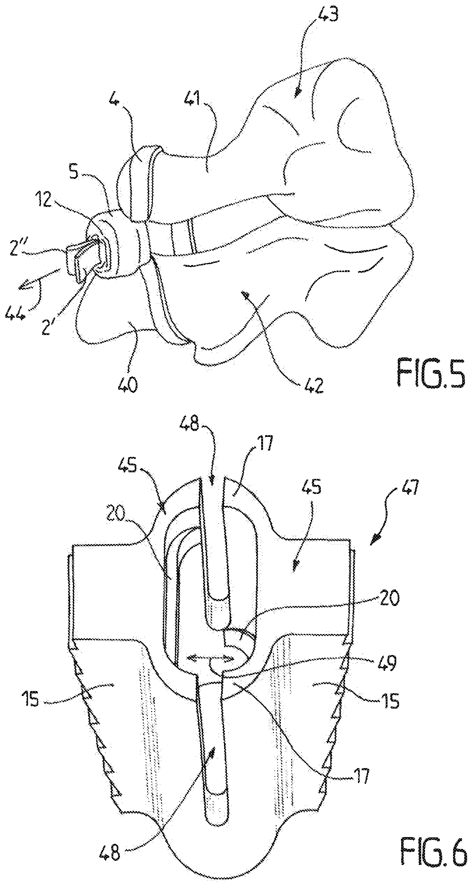

On va maintenant décrire plus particulièrement en référence aux figures 1A, 1B et à la figure 5 la fixation entre elles des parties osseuses 40 et 41 de deux vertèbres 42, 43 par le dispositif selon le mode de réalisation plus particulièrement décrit.

Après avoir passé la bande en boucle autour des deux parties osseuses 40 et 41, le chirurgien introduit les portions d'extrémité 2 et 2' dans l'orifice 11 du corps, puis dans l'alésage 13, et/ou simultanément dans les deux si ces derniers sont déjà pré connectés (sommairement) l'un à l'autre.

Le corps et la pièce sont alors rapprochés des deux parties osseuses avec un outil de serrage de type connu selon la flèche 44. Le frottement entre les extrémités est suffisant pour pré positionner le dispositif avec la boucle et à la distance voulues.

Une fois la position recherchée obtenue par l'opérateur, celui-ci vient alors insérer en force la pièce dans le corps de façon à venir araser la face 45 supérieure de la pièce avec celle 46 du corps en s'appuyant sur les évidements 10.

Cette insertion en force entraine la déformation plastique ou permanente de la pièce.

Les dents de la pièce et du corps sont alors en prise les unes avec les autres et forment un système de blocage ou anti-retour s'opposant à leur désolidarisation.

On a pu constater qu'une pièce en matière polymère permettait de ne pas détériorer la bande et qu'un corps en titane augmentait l'efficacité du serrage.

On coupe ensuite les parties d'extrémité en surplus avant de refermer le dos du patient.

On a représenté sur la figure 6 un autre mode de réalisation d'une pièce 47 selon l'invention dont les deux parois d'extrémité 17 comprennent de plus chacune une fente 48 en vis à vis, sur une hauteur h₁ inférieure à la hauteur H de la pièce, par exemple compris entre 0,5 et 0,8 H.

Chaque fente 48 est traversante de la paroi correspondante 17, et débouche sur la face supérieure 45 de la pièce 47, ce qui va permettre le resserrement de la pièce (flèche 49) lorsqu'il y a enfoncement du coin dans l'orifice du corps.

Les deux fentes 48 permettent de guider la déformation de la pièce 47 et d'éviter les risques de torsion ou de vrillage de la pièce lorsqu'elle est insérée dans le corps.

Ainsi, la pièce comporte des parois pleines ou continues ou encore au plus deux parois comportant une fente, ces deux parois étant en vis-à-vis.

Comme il va de soi et comme il résulte également de ce qu'il précède, la présente invention n'est pas limitée aux modes de réalisation plus particulièrement décrits. Elle en embrasse au contraire toutes les variantes et notamment celles où tous les éléments sont en titane ou au contraire en matière plastique biocompatible.

## Revendications

1. Dispositif (1, 47) de fixation d'un lien sur au moins une partie osseuse (3), comprenant le lien présentant deux portions d'extrémité (2', 2"), un corps (5) de base rigide comportant un orifice (11) traversant, et une pièce (12) de maintien insérable dans ledit orifice,
au moins en partie en forme de coin écrasable comprenant un alésage (13) central de passage desdites portions d'extrémité (2', 2") en vis à vis ledit alésage (13) étant de section transversale déformable entre une première section (S1) de passage libre des portions d'extrémité (2', 2") lorsque la pièce n'a pas été insérée dans l'orifice et une deuxième section (S2) de blocage desdites portions d'extrémité en compression lorsque la pièce est entièrement ou sensiblement entièrement insérée dans l'orifice, **caractérisé en ce que** le lien étant une bande (2) plate et souple, la face interne de l'orifice (11) et la face périphérique externe de la pièce (12) comprennent des moyens (36) de pré connexion de l'un à l'autre par encliquetage.

2. Dispositif selon la revendication 1, caractérisé en ce le corps (5) de base étant allongé autour d'un axe (6), l'orifice (11) présente une première section longitudinale trapézoïdale ou sensiblement trapézoïdale isocèle dans un premier plan axial (18, 35) avec un premier angle à la base a et la pièce (12) de maintien une seconde section longitudinale trapézoïdale ou sensiblement trapézoïdale isocèle dans ledit premier plan (18, 35) axial avec un second angle à la base b > a.

3. Dispositif selon l'une quelconque des revendications précédentes, **caractérisé en ce que** la pièce (12) de maintien comprend deux parois longitudinales opposées principales (15) et deux parois longitudinales opposées d'extrémité (17), chacune des parois longitudinales d'extrémité (17) étant munie d'une fente traversante (48) sur une portion de ladite paroi, ouvert sur l'alésage et débouchant sur la face supérieure (45) de la pièce (2) .

4. Dispositif selon l'une quelconque des revendications précédentes, **caractérisé en ce que** la pièce de maintien (47) comprend deux parois d'extrémité (17) munies chacune d'une fente (48) en vis-à-vis sur une hauteur h1 inférieure à la hauteur H de la pièce.

5. Dispositif selon l'une quelconque des revendications précédentes, **caractérisé en ce que** la face interne de l'orifice (11) du corps (5) et la face périphérique externe de la pièce (12) comportent des moyens constituant un système de blocage de la pièce (12) par rapport au corps (5), lorsque la pièce est insérée dans l'orifice.

6. Dispositif selon la revendication 4 ou 5, **caractérisé en ce que** l'alésage (13) et la pièce (12) présentent chacun un plan (18, 35) de symétrie longitudinal, définissant respectivement deux faces longitudinales internes opposées pour l'orifice et deux faces longitudinales périphériques externes opposées pour la pièce, chaque face opposée comportant au moins une portion munie d'au moins une dent (16, 36) ou nervure d'accrochage et de blocage de la pièce (12) par rapport au corps (5).

7. Dispositif selon l'une quelconque des revendications précédentes, **caractérisé en ce que** l'alésage (13) présente une section transversale de forme oblongue et comporte deux parois principales opposées (19), au moins une paroi principale comprenant sur sa face interne au moins une nervure (20) transversale en saillie vers l'intérieur.

8. Dispositif selon la revendication 6, **caractérisé en ce que** la nervure (20) transversale en saillie est située dans la partie longitudinale supérieure de l'alésage.

9. Dispositif selon l'une quelconque des revendications précédentes, **caractérisé en ce que** l'alésage (13) et la pièce (12) de maintien sont de formes agencées pour coopérer à frottement sur au moins une de leur partie en vis à vis dans le sens perpendiculaire au premier plan longitudinal (18) lorsque la pièce est insérée dans l'orifice.

10. Dispositif selon l'une quelconque des revendications précédentes, **caractérisé en ce que** la pièce (12) présente un noyau évidé par l'alésage (13), de forme générale oblongue, comprenant deux parois (15) principales opposées en biais formant le coin et deux parois (17) d'extrémité allongées autour d'un axe, arrondies, dont les faces sont agencées pour coopérer longitudinalement avec une partie en évidement - correspondante de l'orifice (11), l'épaisseur des jonctions (22) entre parois principales et parois d'extrémité étant amoindrie pour autoriser la déformation par écrasement dans le sens transversal sans déformation dans le sens longitudinal lors de l'insertion de la pièce dans l'orifice.

11. Dispositif selon l'une quelconque des revendications précédentes, **caractérisé en ce que** le corps (5) de base est en titane, la pièce (12) de maintien est en matériau polymère et la bande (2) souple est une tresse en polymère.

## Patentansprüche

1. Vorrichtung (1, 47) zur Befestigung eines Verbindungsglieds an mindestens einem Knochenteil (3), umfassend das Verbindungsglied mit zwei Endabschnitten (2', 2"), einen steifen Grundkörper (5) mit einem Durchgangsloch (11) und ein in das Loch einsetzbare Haltestück (12),
mindestens teilweise in Form einer quetschbaren Ecke mit einer mittigen Bohrung (13) für die Durchführung der gegenüberliegenden Endabschnitte (2', 2"),
wobei die Bohrung (13) einen Querschnitt aufweist, der zwischen einem ersten Abschnitt (S1) für den freien Durchgang der Endabschnitte (2', 2"), wenn das Stück nicht in das Loch eingesetzt worden ist, und einem zweiten Abschnitt (S2) für die Arretierung der gequetschten Endabschnitte, wenn das Stück vollständig oder im Wesentlichen vollständig in das Loch eingesetzt ist, verformbar ist,
**dadurch gekennzeichnet,**
**dass**, das Verbindungsglied ein flaches und flexibles Band (2) ist,
wobei die Innenseite des Lochs (11) und die umfängliche Außenseite des Stücks (12) Mittel (36) für die Vorverbindung miteinander durch Einrasten umfassen.

2. Vorrichtung nach Anspruch 1,
**dadurch gekennzeichnet,**
**dass**, der Grundkörper (5) sich um eine Achse (6) erstreckt,
wobei das Loch (11) einen ersten trapezförmigen bzw. im Wesentlichen trapezförmigen gleichschenkeligen Längsschnitt in einer ersten axialen Ebene (18, 35) mit einem ersten Winkel an der Basis a aufweist und das Haltestück (12) einen zweiten trapezförmigen bzw. im Wesentlichen trapezförmigen gleichschenkeligen Längsschnitt in der ersten axialen Ebene (18, 35) mit einem zweiten Winkel an der Basis b > a aufweist.

3. Vorrichtung nach einem der vorhergehenden Ansprüche,
**dadurch gekennzeichnet,**
**dass** das Haltestück (12) zwei entgegengesetzte längslaufende Hauptwände (15) und zwei entgegengesetzte längslaufende Endwände (17) aufweist,
wobei die jeweiligen längslaufenden Endwände (17) mit einem durchgehenden Schlitz (48) auf einem Abschnitt der Wand versehen sind, welcher zur Bohrung hin offen ist und in der Oberseite (45) des Stücks (12) mündet.

4. Vorrichtung nach einem der vorhergehenden Ansprüche,
**dadurch gekennzeichnet,**
**dass** das Haltestück (47) zwei Endwände (17) aufweist, die jeweils mit einem gegenüberliegenden Schlitz (48) auf einer Höhe h1 versehen sind, die kleiner ist als die Höhe H des Stück.

5. Vorrichtung nach einem der vorhergehenden Ansprüche,
**dadurch gekennzeichnet,**
**dass** die Innenseite des Lochs (11) des Körpers (5) und die umfängliche Außenseite des Stücks (12) Mittel aufweisen, die ein System zur Arretierung des Stücks (12) in Bezug auf den Körper (5) bilden, wenn das Stück in das Loch eingesetzt ist.

6. Vorrichtung nach Anspruch 4 oder 5,
**dadurch gekennzeichnet,**
**dass** die Bohrung (13) und das Stück (12) jeweils eine Längsspiegelebene (18, 35) aufweisen, die jeweils zwei entgegengesetzte längslaufende Innenseiten für das Loch und zwei entgegengesetzte längslaufende umfängliche Außenseiten für das Stück definiert,
wobei jede entgegengesetzte Seite mindestens einen Abschnitt mit mindestens einem Zahn (16, 36) bzw. Rippe zum Einhängen und Arretieren des Stücks (12) in Bezug auf den Körper (5) aufweist.

7. Vorrichtung nach einem der vorhergehenden Ansprüche,
**dadurch gekennzeichnet,**
**dass** die Bohrung (13) einen länglichen Querschnitt aufweist und zwei entgegengesetzte Hauptwände (19) umfasst,
wobei mindestens eine Hauptwand auf ihrer Innenseite mindestens eine querlaufende, nach innen ragende Rippe (20) aufweist.

8. Vorrichtung nach Anspruch 6,
**dadurch gekennzeichnet,**
**dass** die querlaufende vorragende Rippe (20) in dem längslaufenden oberen Bereich der Bohrung angeordnet ist.

9. Vorrichtung nach einem der vorhergehenden Ansprüche,
**dadurch gekennzeichnet,**
**dass** die Bohrung (13) und das Haltestück (12) derart ausgebildet sind,
**dass** sie auf mindestens einem ihres gegenüberliegenden Bereiche senkrecht zu der ersten Längsebene (18) reibschlüssig sind, wenn das Stück in das Loch eingesetzt ist.

10. Vorrichtung nach einem der vorhergehenden Ansprüche,
**dadurch gekennzeichnet,**
**dass** das Stück (12) einen durch die Bohrung (13) ausgehöhlten im Wesentlichen länglichen Kern mit zwei entgegengesetzten schrägen, die Ecke bildenden Hauptwänden (15) und zwei sich um eine Achse erstreckenden abgerundeten Endwänden (17) aufweist, deren Flächen angeordnet sind, um in Längsrichtung mit einer korrespondierenden Ausnehmung des Lochs (11) zusammenzuwirken,
wobei die Dicke der Übergänge (22) zwischen Hauptwänden und Endwänden verringert ist, um die Verformung durch Quetschen in Querrichtung ohne Verformung in Längsrichtung beim Einsetzen des Stücks in das Loch zuzulassen.

11. Vorrichtung nach einem der vorhergehenden Ansprüche,
**dadurch gekennzeichnet,**
**dass** der Grundkörper (5) aus Titan besteht, das Haltestück (12) aus Polymermaterial besteht und das flexible Band (2) ein Polymergeflecht ist.

## Claims

1. Device (1, 47) for fixing a tie on at least one bone part (3), comprising the tie having two end portions (2', 2"), a rigid base body (5) with a through-opening (11), and a retaining component (12) insertable in said opening,
at least partially in the shape of a squeezable wedge comprising a central bore (13) for passage of said opposite end portions (2', 2"), said bore (13) having a cross section that is deformable between a first cross section (S1) for free passage of the end portions (2', 2") when the component has not been inserted in the opening, and a second cross section (S2) for blocking said end portions by compression when the component is entirely or substantially entirely inserted in the opening, **characterized in that**, with the tie being a flat and flexible band (2), the inner face of the opening (11) and the outer peripheral face of the component (12) comprise means (36) for pre-connection to each other by a snap-fit action.

2. Device according to Claim 1, **characterized in that**, with the base body (5) being lengthened about an axis (6), the opening (11) has a first longitudinal cross section that is an isosceles trapezoid or substantially an isosceles trapezoid in a first axial plane (18, 35) with a first angle at the base a, and the retaining component (12) has a second longitudinal cross section that is an isosceles trapezoid or substantially an isosceles trapezoid in said first axial plane (18, 35) with a second angle at the base b > a.

3. Device according to either of the preceding claims, **characterized in that** the retaining component (12) comprises two main opposite longitudinal walls (15) and two opposite longitudinal end walls (17), each of the longitudinal end walls (17) being provided with a through slit (48) which is arranged on a portion of said wall, is open to the bore and opens out on the upper face (45) of the component (12).

4. Device according to any one of the preceding claims, **characterized in that** the retaining component (47) comprises two end walls (17), each of them being provided with a slit (48) over a height h1 less than the height H of the component, both slits facing each other.

5. Device according to any one of the preceding claims, **characterized in that** the inner face of the opening (11) of the body (5) and the outer peripheral face of the component (12) have means constituting a system for blocking the component (12) with respect to the body (5) when the component is inserted in the opening.

6. Device according to Claim 4 or 5, **characterized in that** the bore (13) and the component (12) each have a longitudinal plane of symmetry (18, 35), respectively defining two opposite inner longitudinal faces for the opening and two opposite outer peripheral longitudinal faces for the component, each opposite face having at least one portion provided with at least one tooth (16, 36) or rib for fastening and blocking the component (12) with respect to the body (5).

7. Device according to any one of the preceding claims, **characterized in that** the bore (13) has a cross section of oblong shape and has two opposite main walls (19), at least one main wall comprising, on its inner face, at least one inwardly protruding transverse rib (20) .

8. Device according to Claim 6, **characterized in that** the protruding transverse rib (20) is situated in the upper longitudinal part of the bore.

9. Device according to any one of the preceding claims, **characterized in that** the bore (13) and the retaining component (12) are of shapes that are designed to cooperate by friction on at least one of their opposite parts in the direction perpendicular to the first longitudinal plane (18) when the component is inserted in the opening.

10. Device according to any one of the preceding claims, **characterized in that** the component (12) has a core hollowed out by the bore (13), of general oblong shape, comprising two opposite inclined main walls (15) forming the wedge and two rounded end walls (17) lengthened about an axis, of which the faces are arranged to cooperate longitudinally with a corresponding recessed part of the opening (11), the thickness of the junctions (22) between main walls and end walls being reduced in order to permit the deformation by squeezing in the transverse direction without deformation in the longitudinal direction during insertion of the component into the opening.

11. Device according to any one of the preceding claims, **characterized in that** the base body (5) is made of titanium, the retaining component (12) is made of a polymer material, and the flexible band (2) is a polymer braid.
